# EUROPEAN PATENT SPECIFICATION

(11) **EP 2 382 936 B1**
(45) Date of publication and mention of the grant of the patent: **17.06.2015**
(21) Application number: 11164273.2
(22) Date of filing: 29.04.2011
(51) Int. Cl.: A61B 18/14

(54) **Sealing plate having depressions with ceramic insulation**
Dichtungsplatte mit keramisch isolierten Vertiefungen
Plaque de scellage dotée de dépressions avec isolation céramique

(30) Priority: 29.04.2010 US 770387
(43) Date of publication of application: 02.11.2011
(73) Proprietor: Covidien LP, Mansfield, MA 02048 (US)
(72) Inventor: Horner, Glenn A., Boulder, CO 80304 (US); Oliver, Christina A., Longmont, CO 80504 (US); Brandt, Kim V., Loveland, CO 80537 (US)
(74) Representative: Hoffmann Eitle

(56) References cited:
- EP-A1- 1 681 027
- EP-A1- 1 769 767
- EP-A1- 1 810 625
- EP-A1- 1 886 637
- EP-A1- 1 946 715
- EP-A2- 1 920 725
- EP-A2- 2 105 104
- US-B2- 7 150 097

## Description

### BACKGROUND

### Technical Field

The present disclosure relates to an apparatus for performing an endoscopic electrosurgical procedure. More particularly, the present disclosure relates to an apparatus for performing an endoscopic electrosurgical procedure that employs an endoscopic electrosurgical apparatus that includes an end effector assembly configured for use with variously-sized access ports.

### Background of the Related Art

Electrosurgical apparatuses (e.g., electrosurgical forceps) are well known in the medical arts and typically include a handle, a shaft and an end effector assembly operatively coupled to a distal end of the shaft that is configured to manipulate tissue (e.g., grasp and seal tissue). Electrosurgical forceps utilize both mechanical clamping action and electrical energy to effect homeostasis by heating the tissue and blood vessels to coagulate, cauterize, fuse, seal, cut, desiccate, and/or fulgurate tissue.

As an alternative to open electrosurgical forceps for use with open surgical procedures, many modern surgeons use endoscopes and endoscopic electrosurgical apparatus (e.g., endoscopic forceps) for remotely accessing organs through smaller, puncture-like incisions. As a direct result thereof, patients tend to benefit from less scarring, less pain, and reduced healing time. Typically, the endoscopic forceps are inserted into the patient through one or more various types of cannulas or access ports (typically having an opening that ranges from about five millimeters to about fifteen millimeters) that has been made with a trocar; as can be appreciated, smaller cannulas are usually preferred.

Endoscopic forceps that are configured for use with small cannulas (e.g., cannulas less than five millimeters) may present design challenges for a manufacturer of endoscopic instruments.

Reference is made to US 7,150,097 B2 and EP 1 946 715 A1.

US 7,150,097 B2 forms the basis of the two-part adopted in the independent claims below.

### SUMMARY

The invention is defined by the independent claims below. Dependent claims are directed towards optional features and preferred embodiments.

An end effector assembly includes a pair of opposing jaw members. One or more of the jaw members includes a support base, optionally an electrical jaw lead, a sealing plate, and a ceramic or other electrically insulative layer. The sealing plate may be coupled to the optional electrical jaw lead and is mounted to the support base. The sealing plate includes a tissue engaging surface, an opposing surface, and a series of depressions formed within the opposing surface and projecting from the tissue engaging surface. One or more of the series of depressions may have a cross-sectional area that is one or more of circular in shape, hemispherical in shape, and rectangular in shape when the tissue engaging surface is viewed from above. In embodiments, two or more of the depressions of the series of depressions have different cross-sections. The series of depressions is formed by one or more of stamping, bending, and machining.

The ceramic or other electrically insulative layer is deposited atop each of the series of depressions. The ceramic layer may be vapor deposited onto the series of depressions. The ceramic layer may have a thickness between about 10 angstroms and about 500 angstroms. The combination of the depressions that project from the tissue engaging surface and the ceramic layer form a corresponding series of nonconductive stop members for controlling the separation distance between opposing jaw members when closed about tissue. Preferably, the ceramic layer for each of the series of depression is surrounded by electrically conductive material of the sealing plate when the tissue engaging surface is viewed from above.

In one aspect, a method of manufacturing a sealing plate of an end effector assembly includes providing one or more jaw members having a support base, an electrical jaw lead, and a sealing plate coupled to the electrical jaw lead and mounted to the support base. The sealing plate includes a tissue engaging surface and an opposing surface. The method includes forming a series of depressions within the opposing surface of the sealing plate such that the series of depressions project from the tissue engaging surface. On step includes depositing a ceramic layer atop each of the series of depressions to form a corresponding series of nonconductive stop members for controlling the separation distance between opposing jaw members when closed about tissue. In one manner, the depositing step involves vapor deposition in a high volume vacuum chamber. The forming step may involve one or more of stamping, bending, and machining.

### BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a perspective view of an endoscopic bipolar forceps in accordance with an embodiment of the present disclosure;

Fig. 2 is a perspective view of an open bipolar forceps in accordance with an embodiment of the present disclosure;

Figs. 3A and 3B are perspective views of opposing jaw members according to an embodiment of the present disclosure;

Figs. 4A and 4B are exploded views of the opposing jaw members of Figs. 3A and 3B respectively;

Fig. 5A is a perspective view of a sealing plate according to an embodiment of the present disclosure; and

Fig. 5B is a rear, cross-sectional view of the sealing plate of Fig. 5A.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is farther away from the user. The term "clinician" refers to any medical professional (i.e., doctor, surgeon, nurse, or the like) performing a medical procedure involving the use of embodiments described herein.

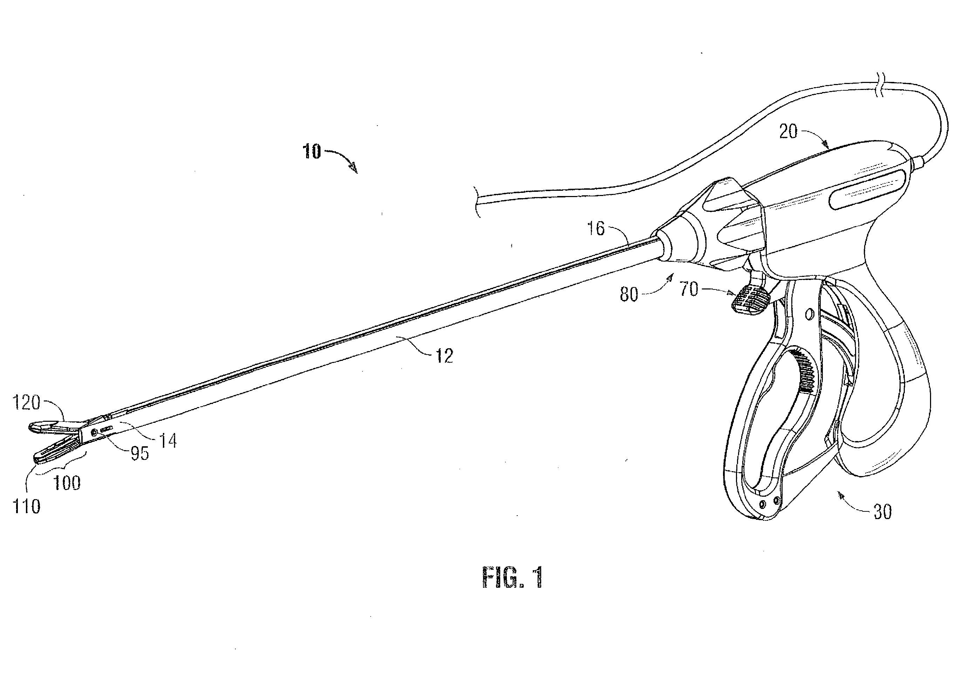

Turning now to Fig. 1, an instrument generally identified as forceps 10 may be used during various surgical procedures and includes a housing 20, a handle assembly 30, a rotating assembly 80, a trigger assembly 70, and an end effector assembly 100 that mutually cooperate to grasp, seal, and divide tubular vessels and vascular tissues. Forceps 10 includes a shaft 12 that has a distal end 14 dimensioned to mechanically engage the end effector assembly 100 and a proximal end 16 that mechanically engages the housing 20. The end effector assembly 100 includes opposing jaw members 110 and 120, which cooperate to effectively grasp tissue for sealing purposes. The jaw members 110 and 120 may be curved to facilitate manipulation of tissue and to provide better "line of sight" for accessing targeted tissues.

Examples of forceps are shown and described in commonly-owned U.S. Application Serial No. 10/369,894 entitled "VESSEL SEALER AND DIVIDER AND METHOD MANUFACTURING SAME" (see US 2003-0229344 A1) and commonly owned U.S. Application Serial No. 10/460,926 (now patent 7,156,846) entitled "VESSEL SEALER AND DIVIDER FOR USE WITH SMALL TROCARS AND CANNULAS (see US 2004-0254573 A1).

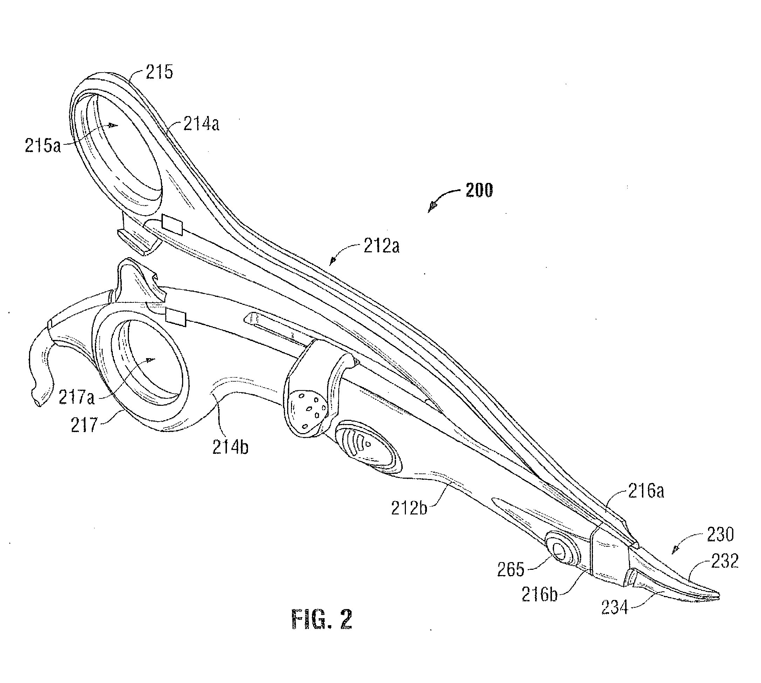

With regard to Fig. 2, an open forceps 200 for use with various surgical procedures is shown. Forceps 200 includes a pair of opposing shafts 212a and 212b having an end effector assembly 230 attached to the distal ends 216a and 216b thereof, respectively. End effector assembly 230 is similar in design to end effector assembly 100 and includes pair of opposing jaw members 232 and 234 that are pivotably connected about a pivot pin 265 and that are movable relative to one another to grasp tissue. Each shaft 212a and 212b includes a handle 215 and 217, respectively, disposed at the proximal end 214a and 214b thereof. Each handle 215 and 217 defines a finger hole 215 and 217a, respectively, therethrough for receiving a finger of the user. Finger holes 215a and 217a facilitate movement of the shafts 212a and 212b relative to one another which, in turn, pivot the jaw members 232 and 234 from an open position wherein the jaw members 232 and 234 are disposed in spaced relation relative to one another to a clamping or closed position wherein the jaw members 232 and 234 cooperate to grasp tissue therebetween.

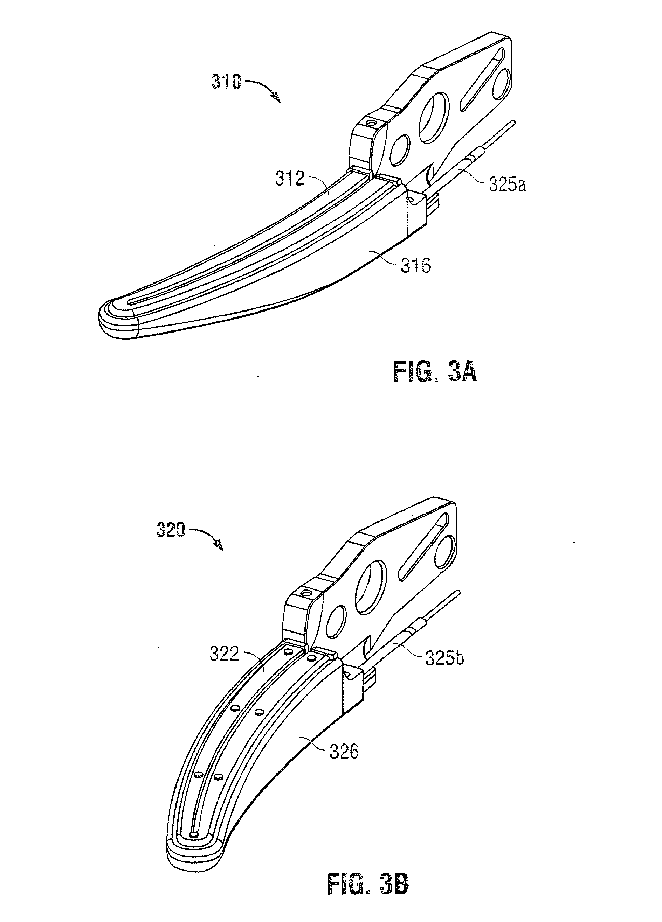

Figs. 3A and 3B are perspective views of opposing jaw members 310 and 320. Similar to jaw members 110 and 120, each of the jaw members 310 and 320 include: sealing plates 312 and 322, respectively; electrical jaw leads 325a and 325b, respectively; and support bases 316 and 326 formed as plastic overmolds. Electrical jaw leads 325a and 325b supply energy to one or both of the opposing jaw members 310 and 320.

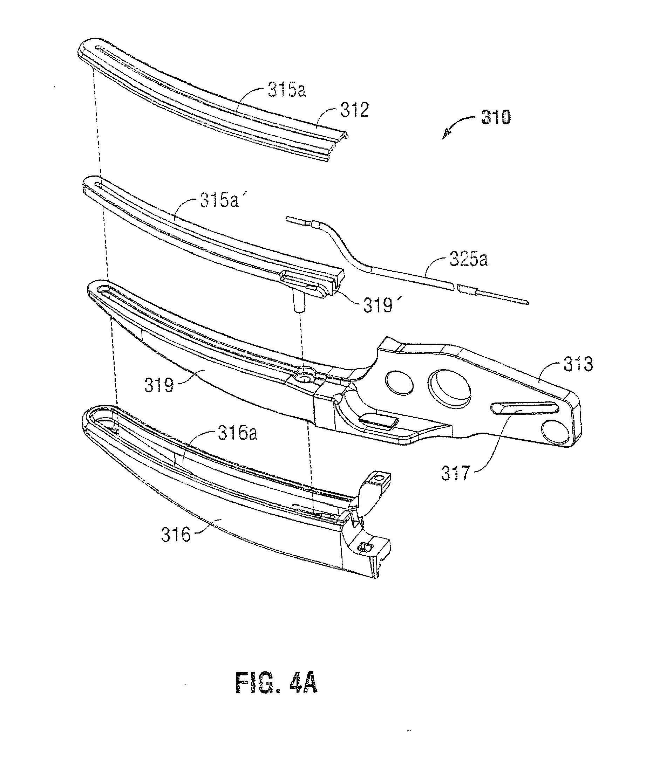

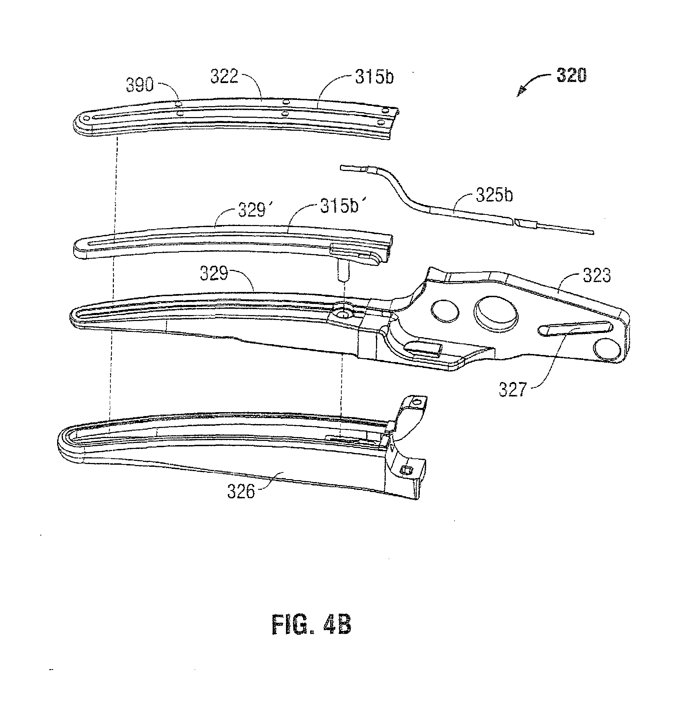

Turning to Figs. 4A and 4B, the opposing jaw members 310 and 320 include support bases 316 and 326 that extend distally from flanges 313 and 323, respectively. The support bases 316 and 326 are dimensioned to support insulative plates 319' and 329', which in turn, support electrically conductive sealing plates 312 and 322 thereon. Sealing plates 312 and 322 may be affixed atop the insulative plates 319' and 329', respectively, and support bases 319 and 329, respectively, in any known manner in the art, snap-fit, over-molding, stamping, ultrasonically welded, etc. The support bases 319 and 329, insulative plates 319' and 329', and sealing plates 312 and 322 are encapsulated by the outer insulative housings 316 and 326 by way of a subsequent overmolding process. The jaw members 310 and 320 are connected via an ultrasonic weld to electrical jaw leads 325a and 325b, respectively.

The jaw members 310 and 320 also include proximal flanges 313 and 323 extending proximally from the support bases 319 and 329, respectively, each of which includes an elongated angled cam slot 317 and 327, respectively, defined therethrough. Jaw member 320 includes a series of stop members 390 disposed on the inner facing surface of electrically conductive sealing plate 312 to define a gap between opposing jaw members 310 and 320 during sealing and cutting of tissue. The series of stop members 390 are applied onto the sealing plate 312 during manufacturing. The electrically conductive sealing plates 312 and 322 and the insulator plates 319' and 329' include respective longitudinally-oriented knife slots 315a, 315a' and 315b, 315b', respectively, defined therethrough for reciprocation of the knife blade (not shown).

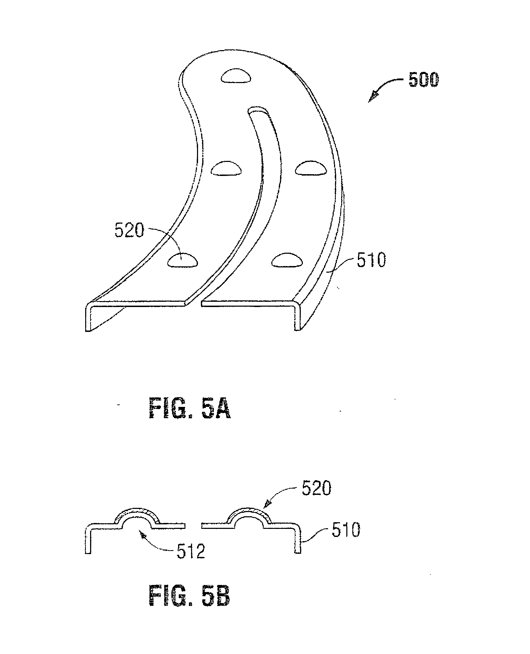

With reference to Fig. 5A, a perspective view of sealing plate 500 is shown. Sealing plate 500 is similar to sealing plate 322 described above. As shown, sealing plate 500 has a stainless steel layer 510 and ceramic layer 520. Like stop members 390, the ceramic layer 520 provides insulation between opposing jaw members 310, 320 (see Figs. 3A and 3B) during sealing and cutting of tissue. Most ceramics are stable at elevated temperatures and usually exhibit low thermal and electrical conductivities, In addition, ceramic materials have high melting points and are resistant to oxidation, corrosion, or other forms of degradation to which metals are usually more prone.

As best shown in Fig. 5B, stainless steel layer 510 includes one or more depressions 512 which may be formed by stamping, a process where metal is formed by being pressed with an embossed pattern, bending, a manufacturing process that produces a V-shape, U-shape, or channel shape along a straight axis in ductile materials, or machining, a material-working processes in which power-driven machine tools, such as lathes, milling machines, and drill presses, are used with a sharp cutting tool to mechanically cut the material to achieve the desired geometry. Accordingly, each depression 512 may be any suitable shape including a shape having circular or non-circular cross-sectional areas. As shown, each depression 512 may be substantially hemispherically shaped. Stainless steel layer 510 may have a polymer coating to prevent corrosion. The polymer coating may be applied by vapor deposition, heat treatment or any other method that may be used to apply a coating to stainless steel layer 510.

Two types of vapor deposition include chemical vapor deposition ("CVD") and physical vapor deposition ("PVD"). In a typical CVD process, the substrate is exposed to one or more volatile precursors, which react and/or decompose on the substrate surface to produce the desired deposit. Frequently, volatile by-products are also produced, which are removed by gas flow through a reaction chamber. PVD is a variety of vacuum deposition and is a general term used to describe any of a variety of methods to deposit thin films by the condensation of a vaporized form of the material onto various surfaces. This coating method involves purely physical processes such as high temperature vacuum evaporation or plasma sputter bombardment.

Ceramic layer 520 may be positioned onto the reverse side or top of depressions 512 of the stainless steel layer 510 by vapor deposition, e.g., CVD or PVD. In this instance, the tissue engaging surface or sealing plate 322 of jaw member 320 includes a series of projections that form a structured bore for the ceramic layer 520. Once the projections are formed, the ceramic layer 520 may be vapor deposited onto the stainless steel layer 510 in a high volume vacuum chamber in order to manufacture sealing plate 500 at a high production rate and reduced expense due to the efficiency associated with vapor deposition. Ceramic layer 520 may have a thickness ranging from 10 angstroms to about 500 angstroms. Sealing plate 500, which includes stainless steel layer 510 and ceramic layer 520, may have a thickness ranging from 0.005 inches to 0.008 inches. The resulting effect is that jaw member 320 (or any of the aforementioned jaw members 120, 220) includes a series of stop members 390 that project from one or both jaw members and maintain a gap of about 0.001 inch to about 0.006 inches therebetween.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawings are presented only to demonstrate certain examples of the disclosure. Other elements, steps, methods and techniques that are insubstantially different from those described above and/or in the appended claims are also intended to be within the scope of the disclosure.

## Claims

1. An end effector assembly (100) including a pair of opposing jaw members (310; 320), at least one of the jaw members comprising:
a support base (316; 326);
an electrical jaw lead (325a; 325b); and
a sealing plate (312; 322; 500) coupled to the electrical jaw lead and mounted to the support base, the sealing plate including a tissue engaging surface and an opposing surface, **characterized by**:
the sealing plate further including a series of depressions (512) formed within the opposing surface and projecting from the tissue engaging surface; and
a ceramic layer (520) deposited atop each of the series of depressions, wherein the combination of the depressions projecting from the tissue engaging surface and their respective ceramic layers form a corresponding series of nonconductive stop members for controlling the separation distance between the pair of opposing jaw members.

2. The end effector assembly according to claim 1, wherein the at least one of the series of depressions has a cross-sectional area that is at least one of circular in shape, hemispherical in shape, and rectangular in shape.

3. The end effector assembly according to claim 1 or 2, wherein at least two of the depressions of the series of depressions have different cross-sections.

4. The end effector assembly of claim 1, 2 or 3, wherein the ceramic layer is vapour deposited onto the series of depressions.

5. The end effector assembly of claim 1, 2, 3 or 4. wherein the series of depressions is formed by at least one of stamping, bending, and machining.

6. The end effector assembly according to claim 1, 2, 3, 4 or 5, wherein the ceramic layer has a thickness between about 10 angstroms and about 500 angstroms.

7. A method of manufacturing a sealing plate of an end effector assembly (100), comprising the steps of:
providing at least one jaw member (310; 320) having a support base (316; 326), an electrical jaw lead (325a; 325b) and a sealing plate coupled to the electrical jaw lead and mounted to the support base, the sealing plate (312; 322; 500) including a tissue engaging surface and an opposing surface, **characterized by**:
forming a series of depressions (512) within the opposing surface of the sealing plate such that the series of depressions project from the tissue engaging surface; and
depositing a ceramic layer (520) atop each of the series of depressions to form a corresponding series of nonconductive stop members for controlling the separation distance between the at least one jaw member and an opposing jaw member.

8. The method according to claim 7 wherein the depositing step comprises vapour deposition in a high volume vacuum chamber.

9. The method according to claim 7 or 8 wherein the forming step comprises at least of stamping, bending, and machining.

## Patentansprüche

1. Endeffektoraufbau (100) einschließlich eines Paars sich gegenüberliegender Backenelemente (310; 320), wobei mindestens eines der Backenelemente aufweist:
ein Stützgrundelement (316; 326);
eine elektrische Backenleitung (325a; 325b); und
eine Versiegelungsplatte (312; 322; 500), die mit der elektrischen Backenleitung gekoppelt ist und an dem Stützgrundelement montiert ist, wobei die Versiegelungsplatte eine Gewebeeingriffsfläche und eine gegenüberliegende Fläche aufweist, **dadurch gekennzeichnet, dass**:
die Versiegelungsplatte ferner eine Reihe von Vertiefungen (512) aufweist, die in der gegenüberliegenden Fläche ausgebildet sind und an der Gewebeeingriffsfläche hervorstehen; und
eine Keramikschicht (520), die auf jeder der Reihe von Vertiefungen aufgebracht ist, wobei die Kombination der Vertiefungen, die von der Gewebeeingriffsfläche hervorstehen, und ihre jeweiligen Keramikschichten eine entsprechende Reihe von nicht leitenden Anschlagelementen für eine Kontrolle des Trennabstands zwischen dem Paar sich gegenüberliegender Backenelemente ausbilden.

2. Endeffektoraufbau nach Anspruch 1, bei dem mindestens eine der Reihe von Vertiefungen einen Querschnittsbereich aufweist, der kreisförmig, halbkugelförmig und/oder rechteckförmig ist.

3. Endeffektoraufbau nach Anspruch 1 oder 2, bei dem mindestens zwei der Vertiefungen der Reihe von Vertiefungen unterschiedliche Querschnitte aufweisen.

4. Endeffektoraufbau nach Anspruch 1, 2 oder 3, bei dem die Keramikschicht auf die Reihe der Vertiefungen aufgedampft ist.

5. Endeffektoraufbau nach Anspruch 1, 2, 3 oder 4, bei dem die Reihe von Vertiefungen durch Stanzen, Biegen und/oder Bearbeiten ausgebildet ist.

6. Endeffektoraufbau nach Anspruch 1, 2, 3, 4 oder 5, bei dem die Keramikschicht eine Dicke zwischen in etwa 10 Å und in etwa 500 Å aufweist.

7. Verfahren zum Herstellen einer Versiegelungsplatte eines Endeffektoraufbaus (100), die Schritte umfassend:
Bereitstellen mindestens eines Backenelements (310; 320), das ein Stützgrundelement (316; 326) aufweist, einer elektrischen Backenleitung (325a, 325b) und einer Versiegelungsplatte, die mit der elektrischen Backenleitung gekoppelt ist und an dem Stützgrundelement montiert ist, wobei die Versiegelungsplatte (312; 322; 500) eine Gewebeeingriffsfläche und eine gegenüberliegende Fläche aufweist, **gekennzeichnet durch**:
Ausbilden einer Reihe von Vertiefungen (512) in der gegenüberliegenden Fläche der Versiegelungsplatte, sodass die Reihe von Vertiefungen von der Gewebeeingriffsfläche hervorstehen; und
Anlagern einer Keramikschicht (520) auf jeder der Reihe von Vertiefungen, um eine entsprechende Reihe nicht leitender Anschlagelemente zum Kontrollieren des Trennabstands zwischen dem mindestens einen Backenelement und einem gegenüberliegenden Backenelement auszubilden.

8. Verfahren nach Anspruch 7, bei dem der Ablagerungsschritt ein aufdampfen in einer hochvolumigen Vakuumkammer umfasst.

9. Verfahren nach Anspruch 7 oder 8, bei dem der Ausbildungsschritt Stanzen, Biegen und/oder Bearbeiten umfasst.

## Revendications

1. Ensemble effecteur terminal (100) incluant une paire d'éléments de mâchoire opposés (310 ; 320), au moins un des éléments de mâchoire comprenant :
une base de support (316 ; 326) ;
un conducteur de mâchoire électrique (325a ; 325b) ; et
une plaque d'obturation (312 ; 322 ; 500) couplée au conducteur de mâchoire électrique et montée sur la base de support, la plaque d'obturation incluant une surface venant en prise avec le tissu et une surface opposée, **caractérisé par** :
la plaque d'obturation incluant en outre une série de creux (512) formés dans la surface opposée et faisant saillie de la surface de mise en prise avec le tissu ; et
une couche céramique (520) déposée sur chacun de la série de creux, où la combinaison des creux faisant saillie de la surface de mise en prise avec le tissu et leurs couches céramiques respectives forment une série correspondante d'éléments d'arrêt non conducteurs pour commander la distance de séparation entre la paire d'éléments de mâchoire opposés.

2. Ensemble effecteur terminal selon la revendication 1, dans lequel ladite au moins une de la série de creux présente une zone en section transversale qui a au moins une parmi une forme circulaire, une forme hémisphérique et une forme rectangulaire.

3. Ensemble effecteur terminal selon la revendication 1 ou 2, dans lequel au moins deux des creux de la série de creux ont des sections transversales différentes.

4. Ensemble effecteur terminal selon la revendication 1, 2 ou 3, dans lequel la couche céramique est déposée en phase vapeur sur la série de creux.

5. Ensemble effecteur terminal selon la revendication 1, 2, 3 ou 4, dans lequel la série de creux est formée par au moins un parmi l'estampage, le pliage et l'usinage.

6. Ensemble effecteur terminal selon la revendication 1, 2, 3, 4 ou 5, dans lequel la couche céramique a une épaisseur entre environ 10 angstroms et environ 500 angstroms.

7. Procédé de fabrication d'une plaque d'obturation d'un ensemble effecteur terminal (100), comprenant les étapes de :
réaliser au moins un élément de mâchoire (310 ; 320) comportant une base de support (316 ; 326), un conducteur de mâchoire électrique (325a, 325b) et une plaque d'obturation couplée au conducteur de mâchoire électrique et montée sur la base de support, la plaque d'obturation (213 ; 322 ; 500) incluant une surface venant en prise avec le tissu et une surface opposée, **caractérisé par** :
former une série de creux (512) dans la surface opposée de la plaque d'obturation de sorte que la série de creux fasse saillie de la surface de mise en prise avec le tissu ; et
déposer une couche céramique (520) sur chacun de la série de creux pour former une série correspondante d'éléments d'arrêt non conducteurs pour commander la distance de séparation entre ledit au moins un élément de mâchoire et un élément de mâchoire opposé.

8. Procédé selon la revendication 7, dans lequel l'étape de dépôt comprend le dépôt en phase vapeur dans un chambre de vide de volume élevé.

9. Procédé selon la revendication 7 ou 8, dans lequel l'étape de formation comprend au moins un parmi l'estampage, le pliage et l'usinage.
